# EUROPÄISCHE PATENTSCHRIFT

(11) **EP 2 163 892 B1**
(45) Veröffentlichungstag und Bekanntmachung des Hinweises auf die Patenterteilung: **16.07.2014**
(21) Anmeldenummer: 08164113.6
(22) Anmeldetag: 11.09.2008
(51) Int. Cl.: G01N 21/85, G01N 33/18, G01N 1/14

(54) **Abwasser-Prozess-Analysegerät**
Wastewater process analyser
Appareil d'analyse de procédé pour des eaux usées

(43) Veröffentlichungstag der Anmeldung: 17.03.2010
(73) Patentinhaber: Hach Lange GmbH, 14163 Berlin (DE)
(72) Erfinder: Gaßner, Bernd, 41469, Neuss (DE)
(74) Vertreter: ter Smitten, Hans

(56) Entgegenhaltungen:
- EP-A- 0 821 231
- WO-A-94/25875
- DE-A1- 10 126 054
- DE-U1- 29 607 783

## Beschreibung

Die Erfindung bezieht sich auf ein Prozess-Analysegerät, wie es beispielsweise in der Abwasser-Analytik in Form einer Tauchsonde eingesetzt wird.

Prözess-Analysegeräte weisen einen oder mehrere Vorratstanks zur Bevorratung von Flüssigkeiten, z.B. Trägerflüssigkeiten oder Reagenzien auf, die für die Durchführung der Analyse benötigt werden. Ferner ist mindestens eine Pumpe zum Pumpen der Flüssigkeit von dem Vorratstank zu dem Analysator, beispielsweise einem Fotometer, vorgesehen. Wenn die Pumpe defekt ist oder ein Vorratstank leer ist, muss das Analysegerät geöffnet werden, um den Vorratstank aufzufüllen beziehungsweise die Pumpe auszutauschen.

Aus DE 101 26 054 A1 ist ein Prozess-Analysegerät bekannt, das modular aufgebaut ist, also aus einem Basismodul und einem Kartuschenmodul besteht. In dem Kartuschenmodul sind Vorratstanks für Flüssigkeiten vorgesehen. Ferner ist in dem Kartuschenmodul auch eine Sonde zur Entnahme einer Flüssigkeitsprobe vorgesehen, die durch eine Pumpenmechanik zu dem Basismodul gepumpt wird. Der Antriebsmotor für die Pumpenmechanik ist auf der Seite des Basismoduls angeordnet.

Aus der Druckschrift WO 94/25875 ist ein Prozess-Analysegerät bekannt, bei dem die Dialysemembran austauschbar ausgebildet ist.

Aufgabe der Erfindung ist es, ein einfach zu wartendes Prozess-Analysegerät zu schaffen.

Diese Aufgabe wird erfindungsgemäß durch ein Prozess-Analysegerät mit den Merkmalen des Patentanspruches 1 gelöst.

Das erfindungsgemäße Prozess-Analysegerät ist als Abwasser-Tauchsonde ausgebildet und modular aufgebaut, und besteht aus einem ständig verwendeten Basismodul und einem austauschbaren Kartuschenmodul.

Das Basismodul weist einen elektrischen Pumpen-Antriebsmotor und ein damit verbundenes Pumpen-Kupplungsteil mit einem Abtrieb auf. Ferner weist das Basismodul einen Analysator auf, beispielsweise ein Fotometer. Der Analysator ist mit einem Leitungs-Kupplungsteil verbunden, durch den eine Flüssigkeit, beispielsweise eine Trägerflüssigkeit oder eine Reagenzflüssigkeit, mittelbar oder unmittelbar zu dem Analysator gelangt. Das Kartuschenmodul weist mindestens einen Vorratstank auf, in dem eine Flüssigkeit gespeichert wird. Der Vorratstank ist verbunden mit einem Leitungs-Kupplungstell, das mit dem basismodulseitigen Leitungs-Kupplungsteil zusammen gekoppelt ist. Zwischen dem Vorratstank und dem Leitungs-Kupplungsteil ist eine antriebslose Pumpenmechanik vorgesehen, die die Flüssigkeit aus dem Vorratstank zu dem Leitungs-Kupplungsteil pumpt. Ferner weist das Kartuschenmodul ein Pumpen-Kupplungsteil mit einem Antrieb auf, das mit dem basismodulseitigen Pumpen-Kupplungsteil verbunden ist. Die Pumpenmechanik des Kartuschenmoduis zu wird also durch den basismodulseitigen Antriebsmotor angetrieben.

In dem Kartuschenmodul sind also nicht nur eine oder mehrere Vorratstanks, sondern auch mindestens eine antriebslose Pumpe vorgesehen. Auf diese Weise wird bei jedem durch die Erschöpfung der Flüssigkeiten in den Vorratstanks veranlassten Austausch des Kartuschenmoduls auch die relativ anfällige Pumpenmechanik ausgetauscht. Der Austausch des Kartuschenmoduls ist sehr einfach und kann auch von ungeschulten Personen vorgenommen werden. Die Anordnung der Pumpenmechanik in der Nähe des Vorratstanks hat den Vorteil, dass die Pumpe als Druckpumpe arbeitet, was erhebliche Vorteile bezüglich der Zuverlässigkeit und in Bezug auf Verstopfungsgefahr hat. Vorzugsweise ist die Pumpenmechanik als Peristaltik ausgebildet. Eine Peristaltik-Pumpenmechanik hat gegenüber einer Piezo-Pumpenmechanik erhebliche Vorteile, die besonders nach dem Austauschen eines Kartuschenmoduls von Bedeutung sind. Insbesondere ein Lufteintrag in einer Leitung stellt für eine peristaltische Pumpenmechanik kein Problem dar, da sie gegen Luft ansaugen kann. Ferner kann bei Verwendung einer peristaltische Pumpenmechanik auf Ventile, insbesondere auf anfällige Rückschlag-Ventile verzichtet werden. Die peristaltische Pumpenmechanik bietet ferner die Möglichkeit, mit einer einzigen peristaltische Pumpenmimik mehrere Leitungen, das heißt, mehrere Flüssigkeiten gleichzeitig pumpen zu können. Schließlich ist die peristaltische Pumpenmechanik eine ausgereifte, preiswerte und äußerst zuverlässig Pumpentechnik.

Die Probenflüssigkeit wird dialytisch aus Abwasser gewonnen. Hierzu weist das Basismodul eine Dialyse-Membran auf.

Gemäß einer bevorzugten Ausgestaltung ist die von den beiden Leitungs-Kupplungsteilen gebildete Leitungskupplung als Nadel-Septum-Kupplung ausgebildet. Die Leitungskupplung wird also von einer sich selbst wiederverschließenden Membran einerseits und einer die Membran durchstechenden Nadel andererseits gebildet. Das Septum ist bevorzugt auf der Kartuschenmodul-Seite angeordnet, so dass bei gelöster Leitungskupplung kein Reagenz aus dem Kartuschenmodul auslaufen kann.

Vorzugsweise weist das Kartuschenmodul mehrere Vorratstanks und korrespondierende Leitungs-Kupplungsteile auf, wobei das Basismodul entsprechende Leitungs-Kupplungstelle vorsieht. Als Vorratstank ist vorliegend ein Tank für eine Reagenz, eine Trägerflüssigkeit, eine Reinigungsflüssigkeit oder auch für Abfall-Flüssigkeit zu verstehen. Gemäß einer bevorzugten Ausgestaltung weist das Kartuschenmodul mehrere voneinander unabhängige Pumpenmechaniken für mehrere Vorratstanks auf. Den Pumpenmechaniken sind jeweils entsprechende Antriebsmotoren in dem Basismodul zugeordnet. Auf diese Weise können mehrere Reagenzien unabhängig voneinander dosiert werden.

Gemäß einer bevorzugten Ausgestaltung weist der Analysator ein Fotometer zur fotometrischen Bestimmung einer Substanz in einer Probenflüssigkeit auf.

Im Folgenden wird die Erfindung anhand einer Zeichnung näher erläutert.

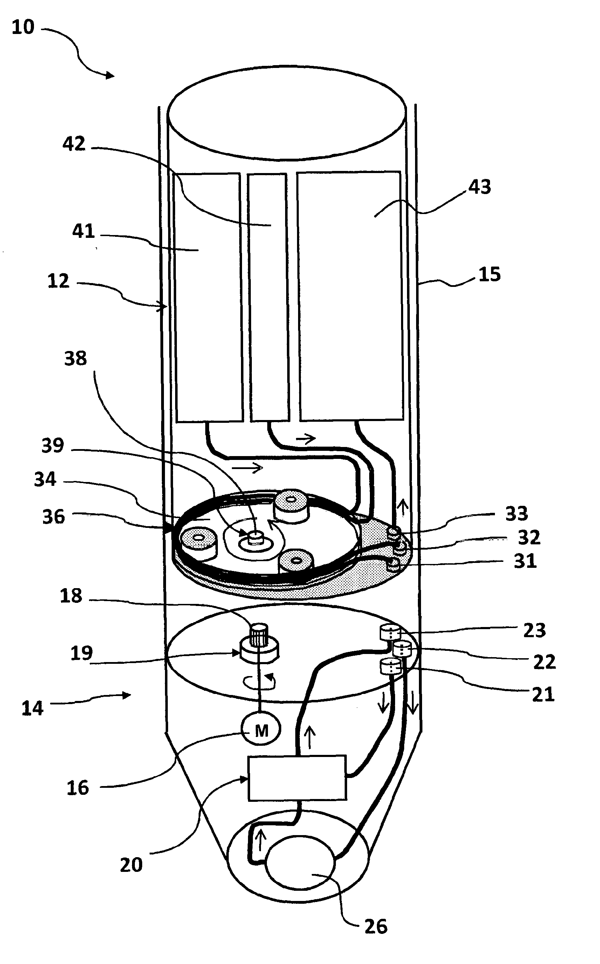

Die Figur zeigt eine schematische Darstellung eines als Tauchsonde ausgebildeten Analysegerätes.

In der Figur ist ein Prozess-Analysegerät 10 dargestellt, das als Tauchsonde ausgebildet ist, und der Analyse von Abwasser zur Regelung eines Klärprozesses dient. Das Analysegerät 10 ist zweiteilig modular aufgebaut, und besteht aus einem Basismodul 14 und einem austauschbaren Kartuschenmodul 12. Das Basismodul 14 weist ein Gehäuse 15 auf, in das das Kartuschenmodul 12 eingesteckt ist.

Das Basismodul 14 weist einen als Fotometer ausgebildeten Analysator 20 auf, der mit einer Flüssigkeitsleitung mit einer ionenselektiven Dialyse-Membran 26 und mit zwei weiteren Flüssigkeitsleitungen mit zwei Leitungs-Kupplungsteilen 22,23 verbunden ist. Eine weitere Flüssigkeitsleitung führt von einem dritten Leitungs-Kupplungsteil 21 zu der Dialyse-Membran 26. Ferner weist das Basismodul 14 einen elektrischen Pumpen-Antriebsmotor 16 auf, der mit einem Abtrieb 18 eines Pumpen-Kupplungsteiles 19 verbunden ist. Der Antriebsmotor 16 ist als Schrittmotor ausgebildet.

Das Kartuschenmodul 12 weist drei Vorratstanks 41,42,43 auf, in denen eine Trägerflüssigkeit, ein Reagenz sowie Abfallflüssigkeit gelagert wird. Der Trägerflüssigkeits-Tank 41 und der Reagenz-Tank 42 sind jeweils über eine Flüssigkeitsleitung an eine einzige gemeinsame peristaltische Pumpenmechanik 36 angeschlossen, durch die die Trägerflüssigkeit beziehungsweise das Reagenz fein dosiert gepumpt werden kann. Die peristaltische Pumpenmechanik 36 ist derart ausgelegt, dass ein unerwünschter Rückfluss oder Leckfluss der Trägerflüssigkeit oder des Reagenz nicht auftreten kann.

Die Flüssigkeitsleitungen des Reagenz-Vorratstanks 42 und des Trägerflüssigkeits-Tanks 41 münden jeweils in ein Leitungs-Kupplungsteil 31,32. Der dritte Vorratstank 43 ist ein Tank für Abfall-Flüssigkeit, in den die von dem Analysator 20 kommende Flüssigkeit fließt. Der dritte Vorratstank 43 mündet in ein drittes Leitungs-Kupplungsteil 33.

Die drei basismodulseitigen Kupplungsteilen 21,22,23 und die drei kartuschenmodulseitigen Leitungs-Kupplungsteile 31,32,33 bilden paarweise jeweils Leitungs-Kupplungen, die als Nadel-Septum-Kupplung ausgebildet sind. Die kartuschenmodulseitigen Kupplungsteile 31,32,33 weisen jeweils das Septum auf, die basismodulseitigen Kupplungsteile 21,22,23 weisen jeweils die Nadel auf.

Der basismodulseitige Pumpen-Antriebsmotor 16 ist über eine von den Kupplungsteilen 19,39 gebildete Antriebs-Kupplung mit der Pumpenmechanik 36 beziehungsweise mit einer rotierenden Pumpenscheibe 34 mechanisch gekoppelt. Hierzu greift ein basismodulseitiges außenverzahntes Abtriebsrad 18 in ein kartuschenmodulseitiges innenverzahntes Antriebrad 38 ein.

Bei Erschöpfung der Reagenz oder der Trägerflüssigkeit, bei drohender Überfüllung des Abfall-Vorratstanks 43 oder bei einer Fehlfunktion, die möglicherweise auf eine der übrigen Komponenten des Kartuschenmoduls 12 zurückgeht, wird das Kartuschenmodul 12 ausgetauscht. Hierdurch wird eine nicht dargestellte Verriegelung entriegelt und das Kartuschenmodul 12 axial nach oben aus dem Basismodul-Gehäuse 15 herausgezogen. Hierbei schließen sich die Septen der kartuschenmodulseitigen Kupplungsteile 31,32,33 und wird die Pumpen-Kupplung gelöst. Anschließend kann ein neues Kartuschenmodul 12 in das Gehäuse 15 eingesetzt werden, wobei die Pumpen-Kupplung geschlossen wird und die Nadeln der basismodulseitigen Kupplungsteile 21, 22, 23 die Septen der kartuschenmodulseitigen Kupplungsteile 31, 32, 33 durchstechen.

Das Kartuschenmodul kann auch mehrere Pumpenmechaniken aufweisen, beispielsweise für jeden Tank eine eigene. Für jede dieser Pumpenmechaniken kann basismodulseitig ein einziger gemeinsamer Antriebsmotor oder aber jeweils ein eigener Antriebsmotor für jeden Tank vorgesehen sein.

## Patentansprüche

1. Abwasser-Prozess-Analysegerät (10) mit einem Basismodul (14) und einem austauschbaren Kartuschenmodul (12), wobei
das Basismodul (14) einen Pumpen-Antriebsmotor (16), ein damit verbundenes Pumpen-Kupplungsteil (19) mit einem Abtrieb (18), einen Analysator (20), ein mit dem Analysator (20) verbundenes Leitungs-Kupplungsteil (21,22) und eine Dialysemembran (26) zur Gewinnung einer Probenflüssigkeit aus Abwasser aufweist,
das Kartuschenmodul (12) einen Flüssigkeits-Vorratstank (41,42), ein mit dem Vorratstank (41,42) verbundenes Leitungs-Kupplungsteil (31,32), das mit dem basismodulseitigen Leitungs-Kupplungsteil (21, 22) eine Leitungskupplung bildet, eine antriebslose Pumpenmechanik (36), die die Flüssigkeit aus dem Vorratstank (41,42) zu dem Leitungs-Kupplungsteil (31,32) pumpt, und ein Pumpen-Kupplungsteil (39) mit einem Antrieb (38) aufweist, das mit dem basismodulseitigen Pumpen-Kupplungsteil (19) verbunden ist, und
das Analysegerät (10) als Abwassär-Täuchsonde ausgebildet ist.

2. Abwasser-Prozess-Analysegerät (10) nach Anspruch 1, wobei die Pumpenmechanik (36) als Peristaltik ausgebildet ist.

3. Abwasser-Prozess-Analysegerät (10) nach Anspruch 1 oder 2, wobei die von den beiden Leitungs-Kupplungsteilen (21,31;22,32) jeweils gebildete Leitungskupplung als Nadel-Septum-Kupplung ausgebildet ist.

4. Abwasser-Prozess-Analysegerät (10) nach einem der vorangegangenen Ansprüche, wobei das Kartuschenmodul (12) mehrere Vorratstanks (41,42) und korrespondierende Leitungs-Kupplungsteile (31,32) aufweist, und das Basismodul (14) entsprechende Leitungs-Kupplungsteile (21,22) aufweist.

5. Abwasser-Prozess-Analysegerät nach einem der vorangegangenen Ansprüche, wobei das Kartuschenmodul mehrere voneinander unabhängige Pumpenmechaniken für mehrere Vorratstanks aufweist.

6. Abwasser-Prozess-Analysegerät (10) nach einem der vorangegangenen Ansprüche, wobei der Analysator (20) ein Fotometer zur fotometrischen Bestimmung einer Substanz in der Probenflüssigkeit aufweist.

## Claims

1. A waste-water process analysis device (10) comprising a base module (14) and an exchangeable cartridge module (12), wherein the base module (14) comprises a pump drive motor (16), a pump coupling member (19) connected thereto and including an output drive unit (18), an analyzer (20), a conduit coupling member (21,22) connected to the analyzer (20), and a dialysis membrane (26) for obtaining a sample liquid from waste water, wherein the cartridge module (12) comprises a liquid supply tank (41,42), a conduit coupling member (31,32) connected to the supply tank (41,42) and forming a conduit coupling with the base-module-side conduit coupling member (21,22), a driveless pump mechanics (36) for pumping the liquid from the supply tank (41,42) to the conduit coupling member (31,32), and a pump coupling member (39) including a drive unit (38) and connected to the base-module-side pump coupling member (19), and wherein the analysis device (10) is designed as a waste-water immersion probe.

2. The waste-water process analysis device (10) according to claim 1, wherein the pump mechanics (36) is formed as a peristaltic structure.

3. The waste-water process analysis device (10) according to claim 1 or 2, wherein the respective conduit coupling formed by the two conduit coupling members (21,31;22,32) is designed as a needle-septum coupling.

4. The waste-water process analysis device (10) according to any one of the preceding claims, wherein the cartridge module (12) comprises a plurality of supply tanks (41,42) and corresponding conduit coupling members (31,32), and the base module (14) comprises corresponding conduit coupling members (21,22).

5. The waste-water process analysis device according to any one of the preceding claims, wherein the cartridge module comprises a plurality of mutually independent pump mechanics for a plurality of supply tanks.

6. The waste-water process analysis device (10) according to any one of the preceding claims, wherein the analyzer (20) comprises a photometer for photometric determination of a substance in the sample liquid.

## Revendications

1. Appareil d'analyse de procédé (10) pour des eaux usées avec un module de base (14) et un module de cartouche (12) échangeable, dans lequel
ledit module de base (14) comprend un moteur d'entrainement de pompe (16), un élément d'accouplement de pompe (19) avec une prise de force (18), un analyseur (20), un élément d'accouplement de conduites (21, 22) lié à l'analyseur (20), et une membrane de dialyse (26) pour l'obtention d'un liquide échantillon à partir des eaux usées,
ledit module de cartouche (12) comprend un réservoir de liquide (41, 42), un élément d'accouplement des conduites (31, 32) lié au réservoir (41, 42) et formant un raccord de conduites avec l'élément d'accouplement de conduites (21, 22), coté module de base, un mécanisme de pompe (36) sans entrainement, qui pompe le liquide à partir du réservoir (41, 42) vers ledit élément d'accouplement de conduites (31, 32), et un élément d'accouplement de pompe (39) ayant un entrainement (38) et lié à l'élément d'accouplement de pompe (19), coté module de base, et
ledit appareil d'analyse (10) est configuré comme sonde à immersion pour des eaux usées.

2. Appareil d'analyse de procédé (10) pour des eaux usées selon la revendication 1, dans lequel le mécanisme de pompe (36) est configuré comme dispositif péristaltique.

3. Appareil d'analyse de procédé (10) pour des eaux usées selon la revendication 1 ou 2, dans lequel ledit raccord de conduites formé respectivement par les deux éléments d'accouplement de conduites (21, 31; 22, 32) est configuré comme raccord aiguille-cloison.

4. Appareil d'analyse de procédé (10) pour des eaux usées selon l'une quelconque des revendications précédentes, dans lequel le module de cartouche (12) comprend plusieurs réservoirs (41, 42) et des éléments d'accouplement de conduites (31, 32) correspondants, et le module de base (14) comprend des éléments d'accouplement de conduites (21, 22) correspondants.

5. Appareil d'analyse de procédé pour des eaux usées selon l'une quelconque des revendications précédentes, dans lequel le module de cartouche comprend plusieurs mécanismes de pompe, qui sont indépendants l'un de l'autre, pour plusieurs réservoirs.

6. Appareil d'analyse de procédé (10) pour des eaux usées selon l'une quelconque des revendications précédentes, dans lequel ledit analyseur (20) comprend un photomètre pour la détermination photométrique d'une substance dans le liquide échantillon.
